# EUROPEAN PATENT APPLICATION

(11) **EP 4 506 340 A1**
(43) Date of publication of application: **12.02.2025**
(21) Application number: 22933797.7
(22) Date of filing: 28.10.2022
(51) Int. Cl.: C07D 233/54, A61K 31/4174, B63B 59/04

(54) **METHOD FOR PRODUCING MEDETOMIDINE AND ITS DERIVATIVES**

(30) Priority: 22.03.2022 RU 2022107497
(71) Applicant: Obshestvo s Ogranichennoj Otvetstvennostju "Vikzdorove Zhivotnyh", Dachnyy poselok Kraskovo, 140050 (RU)
(72) Inventor: OREHOV, Dmitrii Sergeevich, Saratov, 410049 (RU); VIOLIN, Boris Viktorovich, Moscow, 123022 (RU)
(74) Representative: Spengler, Robert
(86) International application number: PCT/RU2022/050341
(87) International publication number: WO 2023/182903

(57) **Abstract**

The invention relates to the field of pharmaceutical chemistry and discloses methods for obtaining medetomidine of formula **2** 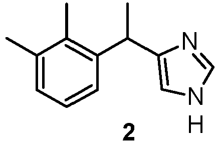 and its derivatives of formula **3** and formula **24** 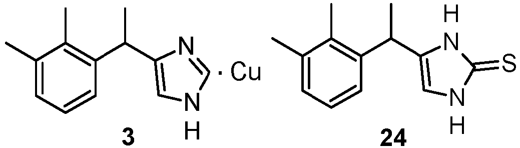 from starting compound **5**

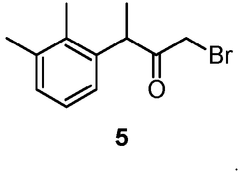

## Description

### Technical field

The invention relates to the field of pharmaceutical chemistry and describes a method for producing medetomidine and its derivatives.

### State of the art

Medetomidine is a synthetic drug which is a racemic mixture of two stereoisomers of 4-[1-(2,3-dimethylphenyl)ethyl]-1H-imidazole. Medetomidine is a potent α2-adrenergic receptor agonist, which causes its dose-dependent analgesic and/or sedative effect. By acting on invertebrate G protein-coupled octopamine receptors, medetomidine inhibits the attachment of mollusks, which is used to prevent biological fouling of the underwater hulls of marine vessels.

Most of the described methods for producing medetomidine are based on the use of precursors already containing an imidazole ring, such as 4(5)-imidazolecarboxylic acid methyl ester (US4544664A, 1985), N-(trimethylsilyl)imidazole (WO2009/53709, 2009; CN105254567, 2016; CN106749027, 2017; CN106588777, 2017; CN111253316, 2020 and CN112979552, 2021), 4(5)-acetylimidazole (CN105884691, 2016), 4(5)-iodimidazole (CN106083724, 2016), (1-trityl-1H-imidazole-4-yl)boronic acid (CN108147999, 2018), 4(5)-imidazolecarbaldehyde (CN107857731, 2018 and KR2021/12112, 2021), 1-dimethylsulfamoyl-2-(tert-butyldimethylsilyl)imidazole (CN109608400, 2019), and 1H-imidazole-4-carbonitrile (WO2021/89878, 2021).

However, the preparation of the above precursors is a separate multi-stage process, often requiring special reaction conditions, since these compounds are not commercially available in most cases.

In particular, patent CN109608400 describes the reaction with n-butyl lithium at -78°C to obtain the key precursor (1-dimethylsulfamoyl-2-(tertbutyldimethylsilyl)imidazole), which requires the use of low-temperature cryogenic units and specially designed reactors.

(1-trityl-1H-imidazol-4-yl)boronic acid used in the method for producing medetomidine described in patent CN108147999 is also not a commercially available precursor, whose preparation is a separate multi-stage technological process.

The disadvantage of the medetomidine synthetic methods based on the interaction of *N*-(trimethylsilyl)imidazole with 1-(1-chloroethyl)-2,3-dimethylbenzene (CN105254567, 2016; CN106749027, 2017; CN111253316, 2020 and CN112979552, 2021), or 1-(2,3-dimethylphenyl)ethanol (WO2009/53709, 2009 and CN106588777, 2017) by the Friedel-Crafts reaction, is the use of a large excess of Lewis acid (titanium tetrachloride in the patents under consideration).

The second synthetic approach to medetomidine production is the creation of an imidazole cycle from precursors which do not initially contain it. In this case, as a rule, linear rather than convergent synthetic schemes are used, and the imidazole ring is usually created at the last stages. In particular, this approach in relation to medetomidine production is described in the present invention.

Currently, there is only little number of documents to disclose this approach in relation to the production of medetomidine. In particular, patents FI77858C (1982), WO2011070069A1 (2011), WO2012/172122 (2012), WO2012/172120 (2012), WO2013/14428 (2013), WO2016/120635 (2016), CN106518812 (2017), and CN111217756 (2020).

One of the first methods for obtaining medetomidine, including the creation of an imidazole ring, is described in patent FI77858C (1982) and is based on the Bredereck reaction (Hellmut Bredereck. Chem. Ber. 1953, 86, 88). Despite its moderate yield (about 30% at the last stage), the reaction is easy to perform and requires no special conditions. However, this patent does not describe any method for the synthesis of the key precursor introduced into the Bredereck reaction, but based on the approaches to the synthesis of α-haloketones known at the publication time of patent FI77858C (excluding direct halogenation), the most widely used method was based on the interaction of diazomethane with carboxylic acid halides, followed by a one-pot reaction of the resulting diazoketone with the corresponding hydrogen halide (Nierenstein M. The action of diazomethane on some aromatic acyl chlorides. J. Chem. Soc. Trans. 1915, 107, 1491-1494). Since diazomethane and diazoketones are extremely explosive and also toxic compounds, the production of medetomidine using this technology has serious disadvantages, in particular, a high explosion hazard occurring when the process is scaled up.

The preparation by the authors of this precursor by direct halogenation of the corresponding carbonyl compound, in our opinion, is excluded, because so far no practical approach to the synthesis of 3-(2,3-dimethylphenyl)-2-butanone 7 has been described in the literature. The only publication where 3-(2,3-dimethylphenyl)-2-butanone 7 was mentioned as a component of a complex, difficult-to-separate mixture of isomeric ketones resulting from the rearrangement of 1,5,8-trimethyl-6-methylidene-tricyclo[3.2.1.0^{2.7}]oct-3-en-8-endo-ol is the paper by Gabriele Mukherjee-Müller et al. (Helv. Chim. Acta 1977, 60, 1758-1780).

Patent WO2011070069A1 (2011) discloses a synthetic method to create an imidazole ring in a multi-step process starting from commercially available 2,3-dimethylbenzoic acid. This method, however, has a significant drawback, which consists in the need to carry out the hydrogenation reaction under pressure (the hydrogenation reaction in this synthesis proceeds in two stages), which requires the use of special equipment (a steel reactor), as well as gaseous hydrogen.

Patents WO2013/14428 (2013) and WO2016/120635 (2016) describe a method for producing medetomidine based on the condensation of 2-bromo-3-(2,3-dimethylphenyl)butanal with formamidine acetate in excess of 25% aqueous ammonia and ethanol. The described method involves few steps, is practical, but also requires a reaction under pressure.

The method for producing medetomidine, including the creation of an imidazole ring, disclosed in patents WO2012/172122 (2012) and WO2012/172120 (2012) and based on the interaction of 2-(2,3-dimethylphenyl)-1-propanal with toluenesulfonylmethyl isocyanide according to the Van Loysen reaction ((a) Albert M. Van Leusen. Tetrahedron Lett. 1972, 2369-2372; (b) J. Org. Chem. 1977, 42, 7, 1153-1159) has significant drawbacks and limitations. In particular, they are the need for chromatographic purification of the medetomidine base, and the use of toxic sodium cyanide. In addition, similarly to patent WO2013/14428 (2013) mentioned above, the reaction leading to the formation of an imidazole ring is also carried out under pressure.

The method for medetomidine synthesis, described in patent CN106518812 (2017), is also based on the Van Loysen reaction and has similar disadvantages to patents WO2012/172122 (2012) and WO2012/172120 (2012), mentioned above.

Thus, there is currently a need to develop novel synthetic routes for the production of medetomidine, which would be safer and fairly cheap when introduced into industrial production. The advantage of the synthetic route developed by us is the wide possibilities for modifying the molecule of the final product (medetomidine) and obtaining various derivatives with biological activity.

In addition, our claimed new method for producing medetomidine and its derivatives compares favorably with those disclosed in patents WO2013/14428 (2013) and WO2016/120635 (2016) in the following parameters:
1. Synthesis of 3-(2,3-dimethylphenyl)-2-butanone 7, the key precursor of medetomidine, is carried out using a one-pot methodology, where the cross-coupling reaction making it possible to significantly complicate the carbon skeleton, and the protective group removal are carried out in one stage, which greatly reduces the total number of synthesis steps. 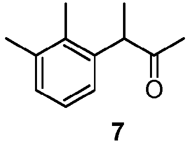
2. The intermediates, namely: 1-bromo-3-(2,3-dimethylphenyl)butan-2-one 5, 3-(2,3-dimethylphenyl)-2-oxobutyl acetate 4, and *N*-[3-(2, 3-dimethylphenyl)-2-oxobutyl]-N-formylformamide 22 can be used in subsequent steps without further purification. 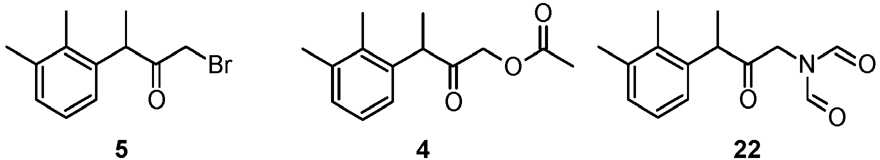
3. Since the described synthetic method is based on the creation of an imidazole ring, there is no need to use hard-to-reach precursors which already contain such a ring.
4. All reactions described in the proposed method for producing medetomidine are carried out under atmospheric pressure, which eliminates the need for special equipment, such as a high-pressure reactor.
5. Any reactions described in the disclosed method for producing medetomidine, requiring cooling or heating, are carried out at moderately low (-15°C) or moderately high temperatures (≤130°C, routes A and B, and ≤160°C, route C), which eliminates the need for special equipment such as low-temperature cryogenic systems and a specially designed reactor.

### SUMMARY OF THE INVENTION

The method according to the present invention is disclosed in the following reaction schemes:

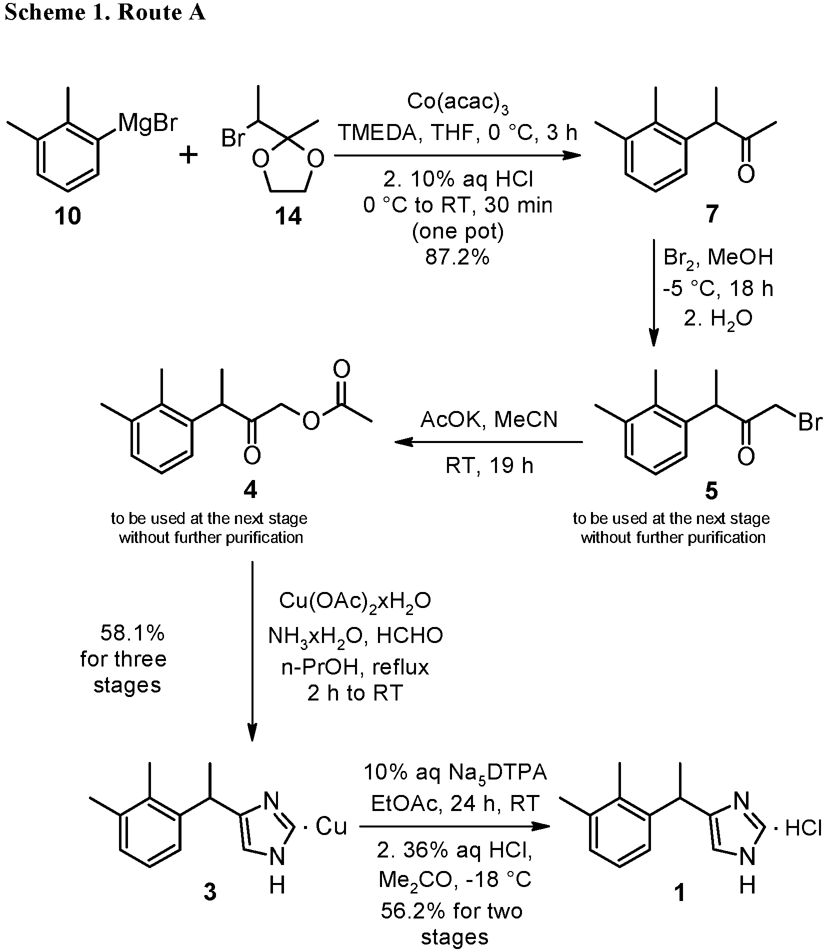

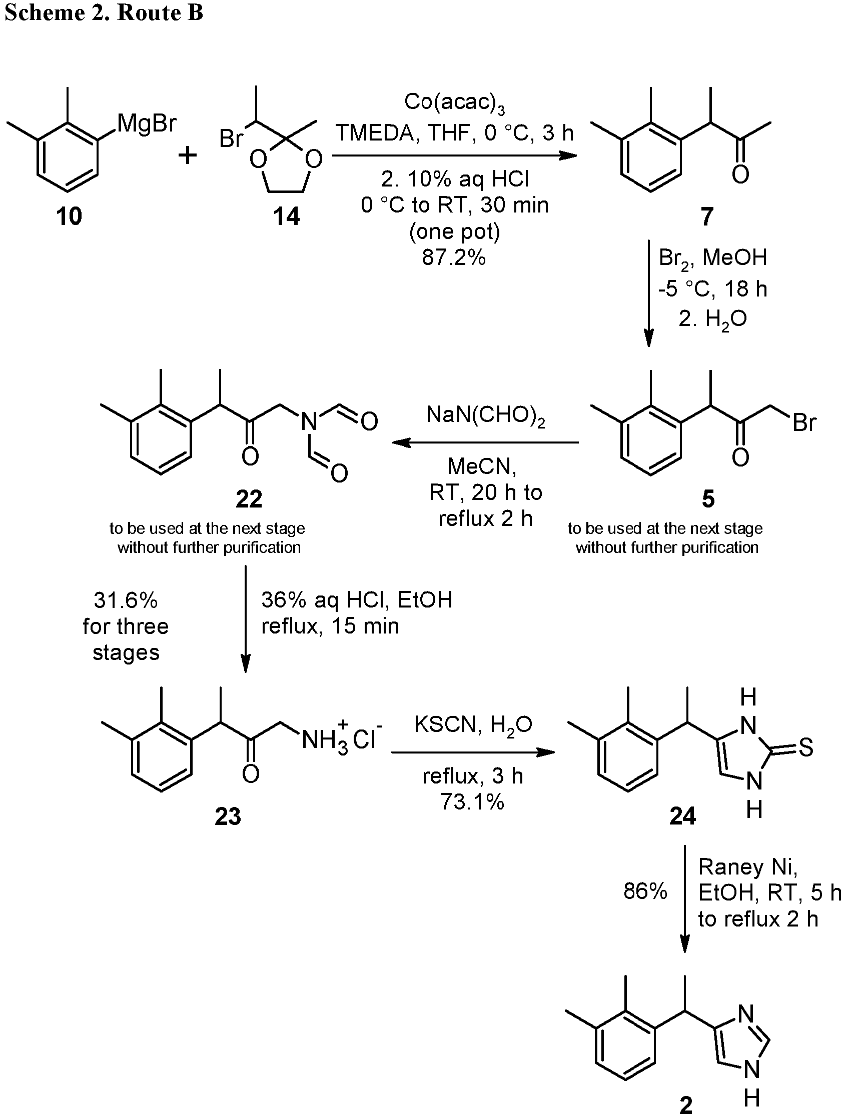

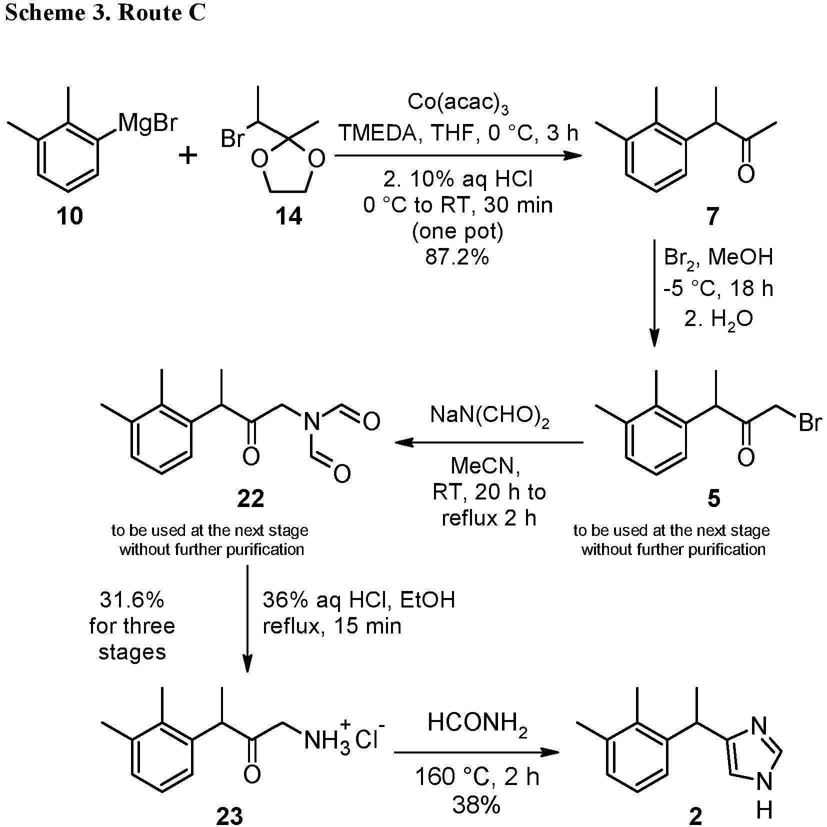

The starting compound for the preparation of 3-(2,3-dimethylphenyl)butan-2-one 7 is commercially available 2,3-dimethylbromobenzene 11 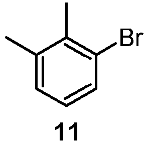 , which can also be obtained from 2,3-dimethylaniline 13 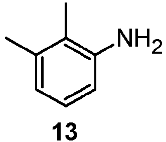 according to literature methods (a modified method from J. Chem. Soc. 1940, 16-18 is described below).

The reaction begins with the production of 2,3-dimethylaniline hydrobromide from 2,3-dimethylaniline **13** and 40% hydrobromic acid. The salt without isolation and purification is introduced into the reaction with nitrous acid generated *in situ* from sodium nitrite and hydrobromic acid taken in excess.

Since the diazotization process proceeds exothermically, it is necessary to carefully control the internal temperature in the reactor, not allowing it to rise above 0°C, which is achieved by changing the rate of addition of the sodium nitrite solution.

Exceeding the specified temperature would lead to a significant drop in the yield of the product, due to the occurrence of side processes and decomposition of the diazonium salt **12** 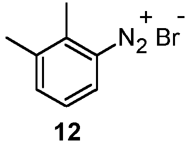 , which is already observed to some extent at 0°C.

Freshly precipitated powdered copper is added to a solution of 2,3-dimethylphenyldiazonium bromide **12,** and the temperature of the solution is raised above 28°C, which causes decomposition of the diazonium salt to release gaseous nitrogen and to form 2,3-dimethylbromobenzene **11.**

Carrying out the process of decomposition of the diazonium salt **12** at a sufficient stirring speed makes it possible to avoid foaming of the reaction mixture due to nitrogen evolution, and to ensure a smooth process.

After spontaneous cessation of temperature rise, the reaction mixture is briefly (20-30 min) heated up to 70-75°C to complete the process, cooled, and extracted with a light organic solvent such as hexane, heptane, or a light fraction of petroleum ether. The use of pentane is not recommended due to its low boiling point, which would lead to significant solvent losses during the processing and recovery process.

Since a significant amount of 2,3-dimethylphenol **25** is formed during diazotization 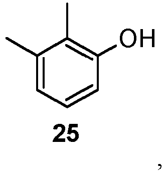 washing the extract with aqueous solutions of strong bases such as sodium hydroxide or potassium hydroxide is a necessary operation. Separation of product **11** from resinous impurities is achieved by steam distillation from an alkaline solution.

The use of heavy solvents (methylene chloride, chloroform) to extract 2,3-dimethylbromobenzene **11** is not recommended, because of their density close to that of the reaction mixture, and also because of their tendency to give a stable emulsion in an alkaline medium, which greatly complicates the process of washing the organic phase with alkaline solutions to remove the impurity of 2,3-dimethylphenol **25.**

The compound of structure **14** 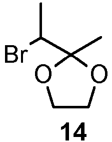 used in the next step can be synthesized from commercially available 3-bromo-2-butanone (structure **15**) 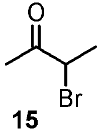 according to the standard procedure described, for example, in Greene's Protective Groups in Organic Synthesis 2006.

The starting 3-bromo-2-butanone **15** can also be obtained by bromination of readily available methyl ethyl ketone **17** 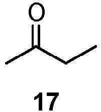 with molecular bromine in an aqueous solution of acetic acid according to literature methods (e.g., Rec. Trav. Chim. Pays-Bas 1946, 65, 691).

Since the bromination reaction of methyl ethyl ketone **17** leads to the formation of two regioisomers, namely, 3-bromo-2-butanone **15** and 1-bromo-2-butanone **16** 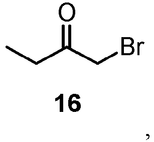 the mixture should be separated by vacuum distillation on an efficient distillation column before the introduction of a protective group. A column 800 mm high and 30 mm in diameter filled with Raschig rings provides 3-bromo-2-butanone **15** containing ≤5% of regioisomer **16** according to NMR. HPLC or GC can also be used to evaluate the **15:16** ratio of regioisomers in the distillate.

The interaction of 3-bromo-2-butanone **15** with ethylene glycol in the presence of catalytic amounts of *p*-toluenesulfonic acid monohydrate in toluene, with simultaneous azeotropic distillation of the water formed during the reaction, leads to the formation of ethylene ketal **14.**

The next stage includes the preparation of 2,3-dimethylphenylmagnesium bromide **10** 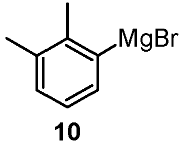 from 2,3-dimethylbromobenzene **11,** the reaction of the obtained organomagnesium reagent with 2-(1-bromoethyl)-2-methyl-1,3-dioxolane **14,** and the removal of the protective group.

The listed reactions are carried out in one pot, without isolation and purification of any intermediate intermediates, and lead to the production of ketone **7**

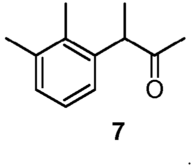

The preparation of the Grignard reagent **10** begins with a direct reaction of 2,3-dimethylbromobenzene **11** and metallic magnesium in dry tetrahydrofuran (THF) at a temperature not exceeding 50°C (optimally 48-50°C). To complete the reaction, short-term heating (70-75°C, 45-60 min) is required.

Solvent (THF) pretreatment involves distillation under argon atmosphere and drying with activated 3A molecular sieves. To remove hydroperoxides from THF, standard methods described in the literature can be used, such as sorption with activated alumina, treatment with Dowex-1 anion exchange resin, and distillation with the preliminary addition of hydroquinone ((a) Zakharov L.N. Safety in chemical laboratories 1991; (b) Donald E. Clark. Peroxides and peroxide-forming compounds. Chem. Health Saf. 2001, 8, 5, 12-22).

The optimal concentration of 2,3-dimethylphenylmagnesium bromide **10** in the solution is 0.8-1 M. Above this concentration, the solution viscosity increases, which complicates the process of its addition during cross-coupling.

Analysis of the concentration and yield of 2,3-dimethylphenylmagnesium bromide 10 in the prepared solution can be performed by titrating an accurate weight of iodine dissolved in a saturated solution of lithium chloride in THF until the brown color of the solution disappears (Paul Knochel. Convenient Titration Method for Organometallic Zinc, Magnesium, and Lanthanide Reagents. Synthesis 2006, 5, 890-891).

Analysis of the concentration of the organomagnesium reagent in the solution by estimating the conversion degree of the starting 2,3-dimethylbromobenzene **11** using GC, followed by extrapolation of the data obtained, cannot be regarded as a correct method for assessing the yield of the organomagnesium reagent, due to the occurrence of a side reaction to form biphenyl **9**

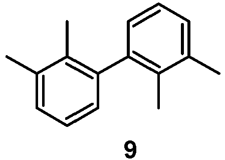

The reaction of 2,3-dimethylphenylmagnesium bromide **10** with 2-(1-bromoethyl)-2-methyl-1,3-dioxolane **14** in the presence of 10 mol% cobalt acetylacetonate (III) and 10 mol% N,N,N',N'-tetramethylethylenediamine (TMEDA) in THF at 0°C leads to the formation of a new C-C bond, and the formation of a cyclic ketal **8**

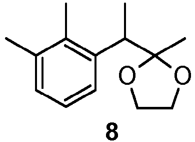

Cyclic ketal **8** is readily cleaved in an acidic medium (10% hydrochloric acid, hydrolysis rate <5 min, UPLC monitoring), which makes it possible to carry out the deprotection process simultaneously with quenching the reaction mixture.

As a result of optimization of the cross-coupling reaction, it was found that a decrease in the amount of catalyst from 10 to 5 mol% leads to a decrease in the yield of ketone 7 from 87.2 down to 60%.

After extraction with a non-polar solvent (heptane) and concentration in vacuum, ketone **7** contains a significant amount (≥20% according to NMR data) of ethylene glycol, which is formed as a result of the cleavage of the protecting group and is distilled in vacuum along with the product, which leads to a broadening of the distillation temperature range to 10°C, and as a result, to a decreased yield, due to the need to separate a larger amount of early fractions, before the start of collecting the major fraction.

Removal of ethylene glycol from 3-(2,3-dimethylphenyl)butan-2-one 7 before vacuum distillation is carried out by repeated washing of the product with water. Because of the density of ketone 7, which is close to that of water, the washing process is complicated by the formation of a stable emulsion. Preliminary dilution of crude ketone **7** with hexane, or another light non-polar solvent (-1:1.5 vol.), makes it possible to completely avoid the formation of any emulsion during the washing process. The completeness of removal of the ethylene glycol impurity is controlled by NMR spectroscopy, by the disappearance of the signal of methylene groups (3.76 ppm, s, CDCl₃), or by GC.

The second by-product formed during the cross-coupling reaction is 2,2',3,3'-tetramethylbiphenyl **9,** which must also be separated before the distillation process, since it is volatile in vacuum and serves as a source of contamination of the target ketone **7** at the end of the distillation process.

Efficient removal of most of 2,2',3,3'-tetramethylbiphenyl (>95% by HPLC) is achieved by diluting crude ketone 7 with methanol (1: 1 vol.), followed by crystallization of biphenyl **9** at -15...-20°C for 2 days. The crystallization time can be reduced to about 20 hours by introducing the crystalline compound **9** into the solution obtained from the still residue as a seed.

Removal of traces of cobalt compounds can be carried out by sorption of the latter by activated (120°C, 20 h) silica gel with particle sizes of 40-63 µm in a non-polar organic solvent such as hexane.

At the next stage, the resulting 3-(2,3-dimethylphenyl)butan-2-one **7** is brominated with molecular bromine at the terminal methyl group to form 1-bromo-3-(2,3-dimethylphenyl)butan-2-one **5**

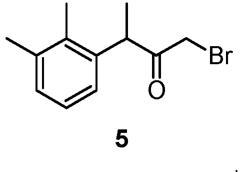

The optimal solvent providing the highest regioselectivity is methanol. The bromination process proceeds within a wide temperature range (from -15°C to room temperature), however, the amount of by-products (di- and tribromo derivatives) formed during the reaction, as well as its rate are extremely dependent on both the temperature and the concentration of the starting ketone in solution.

Bromination at room temperature (20-25°C) proceeds already at the instant of adding bromine, and ends in a few minutes. However, with this mode of bromination, the amount of impurities of polybrominated products greatly increases (HPLC area of the target product **5** ≤49%).

When carrying out bromination at -12°C, the formation rate of polybrominated impurities is significantly slowed down (HPLC area of **5** 81%, HPLC area of dibro-moketone <5%), however, it takes about 28 hours to achieve the conversion degree of the starting ketone >95% (HPLC).

The optimal temperature range for bromination is from -5 to -8°C.

The use of the stoichiometric ratio of bromine : ketone **7** leads to only ≤85% conversion of the latter (HPLC). To achieve complete conversion of the starting ketone, at least 1.15 equivalents of bromine is required, optimally 1.20-1.25 equivalents of bromine.

Control over the course of side processes is also achieved by the degree of dilution of the solution. The use of less than 10 ml of the solvent per 1 g of ketone leads to a sharp increase in side processes accompanied by the formation of polybrominated impurities. The optimal concentration of ketone 7 in solution is in the range of 0.18-0.22 mol/L.

The addition of bromine to the methanolic solution of the ketone should be carried out as quickly as possible, preferably in one portion, since with slow addition, or dropwise addition, a noticeable amount (>10% by HPLC area) of an impurity of the regioisomeric bromination product **20** is formed

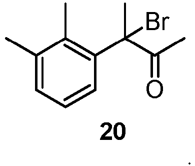

To complete the reaction, it may be necessary to increase the temperature up to 0°C (after about 17-18 hours) and hold at this temperature until the bromine color disappears completely. In case of incomplete conversion of the starting ketone **7,** the introduction of additional bromine portions could be required, which allows achieving >97% (HPLC) conversion of compound 7.

1-Bromo-3-(2,3-dimethylphenyl)butan-2-one dimethyl acetal 6 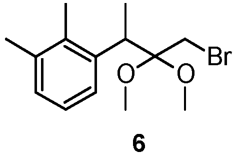 formed during bromination easily cleaved in one pot without isolation (hydrolysis rate at pH 3 <2 min, UPLC) by excessive hydrogen bromide contained in the reaction mixture after its dilution with water, resulting in the formation of 1-bromo-3-(2,3-dimethylphenyl)butane-2-one **5,** which is used in the next step without further purification. Product **5** is not stable at room temperature and darkens quickly, but can be stored at -18°C for several weeks without significant degradation.

The use of acetic acid, esters such as ethyl acetate or halogenated hydrocarbons as solvents leads to the formation of predominantly the most substituted 3-bromo-3-(2,3-dimethylphenyl)butan-2-one **20.**

The use of pyridine hydrobromide perbromide (PHBP, **19**) in THF, ethyl acetate, or glacial acetic acid as a brominating agent also results in almost exclusively 3-bromo-3-(2,3-dimethylphenyl)butan-2-one **20,** and a small amount of the impurity of polybrominated compounds.

3-(2,3-dimethylphenyl)-2-oxobutyl acetate (structure **4**) 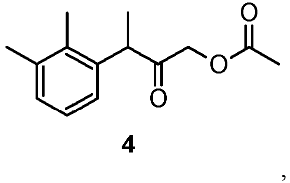 which is a precursor for the formation of the imidazole ring in the medetomidine molecule, in the case of synthesis along route A (Scheme 1), is prepared by reacting 1-bromo-3-(2,3-dimethylphenyl)butan-2-one **5** with potassium acetate (~3 eq) in a suitable organic solvent (acetonitrile, DMF or DMSO) at room temperature.

In the case of using low-boiling solvents (such as acetonitrile), compound **4** can be isolated from the reaction mixture by simply concentrating the solution in vacuum, after pre-filtering the precipitate of potassium bromide and excess potassium acetate.

Removal of acetonitrile traces is achieved by washing the solution of compound **4** in a non-polar solvent (hexane, heptane, MTBE, ethyl acetate, etc.) with water, followed by concentration. The resulting 3-(2,3-dimethylphenyl)-2-oxobutyl acetate **4** is quite pure and can be used at the next stage of the synthesis without additional purification.

Acetonitrile regenerated (>90%) during processing of the reaction mixture can be used without additional preparation at the next loading (at this stage of synthesis), without deteriorating the quality of product **4.**

Carrying out the reaction in methanol at room temperature leads to a low conversion of the starting 1-bromo-3-(2,3-dimethylphenyl)butan-2-one (<60% in 43.5 h). Increasing the reaction temperature in the case of using methanol as a solvent to increase the conversion leads to a sharp rise in side processes, which gives a product unsuitable for use in the next stage without additional purification.

The advantage of carrying out the reaction in DMF or DMSO is the reduction of the reaction time from 18-20 hours (in the case of acetonitrile) to 5-6 hours (DMF); however, in this case, dilution of the reaction mixture with a large volume of water (3-5 vol.) is required for isolation of the product, followed by extraction of the product with a low-boiling organic solvent (hexane, heptane, MTBE, ethyl acetate, etc.).

The final stage of the process includes the formation of an imidazole ring, with the formation of medetomidine as copper (I) complex **3** 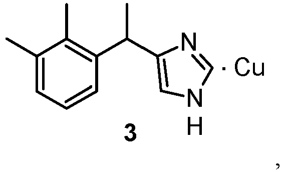 isolation of the free base of medetomidine **2** 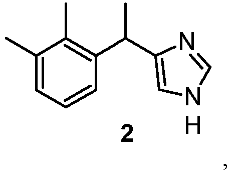 removing copper traces, and obtaining a pharmaceutically acceptable salt such as medetomidine hydrochloride **1**

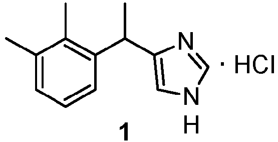

The interaction of 3-(2,3-dimethylphenyl)-2-oxobutyl acetate **4** with formaldehyde, ammonia and copper (II) acetate in a water-alcohol solution according to the Weidenhagen reaction (Weidenhagen R. Chem. Ber. 1935, 68, 1953) leads to the formation of the copper (I) complex of medetomidine **3.**

The optimal alcoholic co-solvent for carrying out the reaction is *n*-propanol, which provides complete solubility of the starting 3-(2,3-dimethylphenyl)-2-oxobutyl acetate **4** in aqueous ammonia solution. The optimal amount of *n*-propanol is from 10 to 15 ml per 1 g of 3-(2,3-dimethylphenyl)-2-oxobutyl acetate **4.** In this case, the precipitation of the copper (I) complex of medetomidine during the reaction occurs in the form of a well-formed crystalline precipitate.

The use of a smaller volume of *n*-propanol would lead to incomplete dissolution of compound **4** and, as a consequence, to the formation of an emulsion, which in turn would lead to the precipitation of the copper complex of medetomidine on the walls of the reactor and on the stirrer in the form of a dense deposit, which complicates the filtration process. In addition, due to the reaction proceeding on the surface of droplets rather than in a homogeneous medium, there is an incomplete conversion of the starting compound **4,** and, as a result, a drop in the yield of the product and its contamination with an unreacted precursor.

The use of a larger volume of *n*-propanol is impractical due to the solubility of complex **3,** which could lead to a decrease in its yield. The reaction proceeds at the boiling point of the solution, and ends in 2 hours. A shorter heating time (<1.5 h) results in a drop in the yield of copper complex **3** from 80.9 down to 58.5% at this stage. The use of methanol, ethanol or isopropanol as co-solvents also leads to precipitation of the copper complex of medetomidine on the walls of the reactor, due to their insufficient dissolving power for compound **4.**

The traditional method for isolating free imidazoles from their copper complexes is precipitating copper as copper (I) sulfide under the action of hydrogen sulfide gas. However, this method cannot be considered as a scalable industrially acceptable process due to the high toxicity of hydrogen sulfide.

The method disclosed by us avoids the use of hydrogen sulfide and is based on the use of non-toxic chelators.

The optimal chelating agent is the pentasodium salt of diethylenetriamine pentaacetic acid (NasDTPA). The optimal molar metal : chelator ratio in this case is in the range of 1 : 1.2-1.5.

The tetrasodium salt of diethylenetriamine tetraacetic acid (Na₄EDTA) has insufficient chelating ability for the copper (I) complex of medetomidine, which leads to low conversion of the starting complex and incomplete release of medetomidine base even when using a 3-5-fold molar excess of the chelating agent and a long reaction time (>4 days).

The equilibrium shift of the process is also achieved by continuous extraction of the medetomidine base into the organic phase, which facilitates the reaction due to the constant removal of the product from the surface of the copper complex crystals. To accelerate the reaction, it is necessary to ensure thorough mixing of the phases.

The use of free acids, such as EDTA or DTPA, as well as their incompletely substituted salts, such as the disodium salt of ethylenediaminetetraacetic acid (Na₂EDTA), as chelating reagents to isolate the free base of medetomidine **2** from the copper complex **3,** leads to the formation of resinous products of complex composition, probably due to the presence of free carboxyl groups simultaneously interacting with the basic nitrogen atom of the imidazole ring and the copper ion.

A pharmaceutically acceptable salt such as medetomidine hydrochloride **1** is formed by reacting medetomidine base **2** with an acid such as hydrochloric acid in a suitable organic solvent. Problems with crystallization of the product may occur due to the high solubility of medetomidine hydrochloride **1** in water, when using concentrated (35-38%) hydrochloric acid in acetone. In this case, to initiate crystallization, it is necessary to add a seed, in the form of a small amount of pure medetomidine hydrochloride crystals. Seeding at the stage of salt formation favorably affects crystallization, which occurs immediately, or almost immediately after the addition of concentrated hydrochloric acid. It takes about a day to complete the crystallization process of compound **1.** The yield of medetomidine hydrochloride can be increased from 56.2 up to 60-65% (based on 2 stages) by concentrating the mother liquor (up to approx. 1/3 of its initial volume) after filtering off the main portion of the crystals, followed by cooling the water-acetone solution for 20-25 hours in a freezer.

Replacing formaldehyde at the stage of formation of the copper complex with any other aldehyde is a convenient way to obtain medetomidine derivatives having a substituent in position 2 of the imidazole ring.

An alternative method to form an imidazole fragment in the medetomidine molecule is route **B** (Scheme 2).

In this case, 1-bromo-3-(2,3-dimethylphenyl)butan-2-one **5** reacts with sodium diformylamide **21** 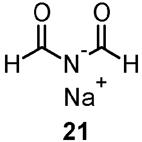 in a suitable organic solvent such as acetonitrile to give intermediate **22** 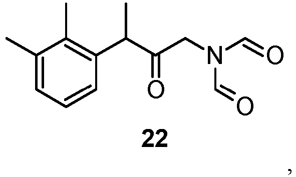 which can be used in the next step without further purification.

The starting, commercially available sodium diformylamide **21** used in the synthesis of compound **22** can also be obtained by literature methods from sodium methoxide and formamide in high yield (e.g., (a) Allenstein E. Chem. Ber. 1967, 100, 3551; (b) US5599986A).

Acidic hydrolysis of compound **22** leads to the formation of aminoketone hydrochloride **23**

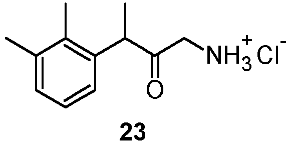

The final step of the process (Route B, Scheme 2) involves the formation of a 2-mercapto-substituted imidazole ring, followed by desulfurization leading to the formation of medetomidine **2.**

The interaction of 1-amino-3-(2,3-dimethylphenyl)butan-2-one hydrochloride **23** with potassium thiocyanate in aqueous solution according to the Marckwald reaction (Marckwald W. Ber. Dtsch. Chem. Ges. 1892, 25, 2354) leads to the formation of 4-[1-(2,3-dimethylphenyl)ethyl]-1,3-dihydro-2H-imidazole-2-thione **24**

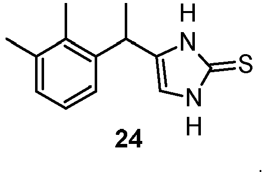

The reaction proceeds smoothly when aminoketone hydrochloride **23** is heated with an excess (4-5 eq) of potassium thiocyanate in an aqueous solution at the boiling point. The optimum heating time is 2.5 to 3.5 hours. The reaction product **24** is almost insoluble in water, in contrast to the starting reagents, and crystallizes out of the solution as the reaction progresses, which greatly simplifies the process of its isolation. If necessary, 4-[1-(2,3-dimethylphenyl)ethyl]-1,3-dihydro-2H-imidazole-2-thione **24** can be purified by crystallization from aqueous ethanol (-65% v/v).

Gentle removal of the mercapto group in compound **24** using Raney nickel in an alcoholic medium leads to the formation of medetomidine **2** in high yield. The best results are obtained using Raney nickel grades W-4 and W-5 (see Robert L. Augustine. Heterogeneous Catalysis for the Synthetic Chemist 1996 for a classification).

The creation of an imidazole ring in the medetomidine molecule can also be performed directly in one step by reacting 1-amino-3-(2,3-dimethylphenyl)butan-2-one hydrochloride **23** with formamide (Scheme 3, Route C). Despite a moderate yield (-40%), the reaction is very easy to perform. Since formamide is taken in excess (-10 eq) and acts in the reaction both as a solvent and as a reagent, and also has a high boiling point (210°C), before extracting the medetomidine base from the reaction mixture, it is advisable to hydrolyze the excessive formamide using concentrated hydrochloric acid (1 hour, room temperature).

### Experimental

### List of abbreviations

| **Abbreviation** | **Meaning** |
|---|---|
| AAS | Atomic absorption spectroscopy |
| acac | Acetylacetonate |
| AcOH | Acetic acid |
| AcOK | Potassium acetate |
| aq | Aqueous solution |
| Area | Chromatographic peak area |
| d | Doublet |
| dd | Doublet of doublets |
| DMF | *N*,*N*-Dimethylformamide |
| DMSO | Dimethyl sulfoxide |
| EtOAc | Ethyl acetate |
| EtOH | Ethanol |
| GC | Gas chromatography |
| HPLC | High Performance Liquid Chromatography |
| m | Multiplet |
| Me₂CO | Acetone |
| MeCN | Acetonitrile |
| MeOH | Methanol |
| MeONa | Sodium methylate |
| MTBE | Methyl *tert*-butyl ether |
| Na₂EDTA | Disodium salt of ethylenediaminetetraacetic acid |
| Na₄EDTA | Tetrasodium salt of ethylenediaminetetraacetic acid |
| NasDTPA | Pentasodium salt of diethylenetriaminepentaacetic acid |
| NMR | Nuclear magnetic resonance |
| n-PrOH | *n-*propanol |
| One pot | One-pot synthesis |
| PHBP | Pyridine hydrobromide perbromide |
| PhMe | Toluene |
| p-TsOH·H₂O | Para-toluenesulfonic acid monohydrate |
| q | Quadruplet |
| Raney Ni | Raney Nickel |
| Reflux | Refluxing |
| Rf | Retention ratio |
| RT | Room temperature |
| s | Singlet |
| THF | Tetrahydrofuran |
| TMEDA | N,N,N',N'-Tetramethylethylenediamine |
| tR | Retention time |
| UPLC | Ultra performance liquid chromatography |

### General methods

The starting 2,3-dimethylaniline **13** was purchased from Sigma-Aldrich (99%, CAS 87-59-2) and used without further purification. All experiments wherein an inert atmosphere was used were performed according to the standard technique using a Schlenk line. Argon 99.999% (Linde Gas) was used as an inert gas without additional purification. All solvents and reagents were purchased from commercial suppliers and used without further purification or preparation, except for THF and methanol. THF (ACS 99.6%) was distilled in an argon atmosphere (with the addition of -0.1% hydroquinone) before use, and dried on activated 3A molecular sieves (100 g/l) for 65 hours. The methanol used in the bromination reaction was dried with activated 3A molecular sieves (45 g/l) for 168 hours. The molecular sieves were activated by heating in vacuum (0.01 mmHg) at 300-320°C for 3 hours. To synthesize the organomagnesium reagent, magnesium shavings of Turnings for Grignard Reaction brand from Fisher Chemical were used, which were stored in an oven at 120°C. Analysis of 2,3-dimethylphenylmagnesium bromide was carried out by direct iodometric titration in a saturated solution of lithium chloride in dry THF according to Paul Knochel's method. Parts of glass facilities (except for large-volume reactors) used at the stage of preparation of organomagnesium reagent **10** and cross-coupling were dried in an oven at 120°C for 24 hours. Glass reactors were dried before handling moisture-sensitive compounds in a rotary vane pump vacuum (0.01 mmHg) by heating with at 90°C for 2 hours, and filled with argon in three pumping/filling cycles. 1H and 13C NMR spectra were recorded on an Agilent 400 MHz spectrometer (400 MHz for 1H and 100 MHz for 13C) in CDCl₃ and D₆-DMSO, respectively. Chemical shifts are expressed in ppm relative to the residual signals of the deuterated solvents used. Analysis of the residual content of heavy metals was carried out on an Agilent Technologies 240 AA atomic absorption spectrometer. A MARS One microwave decomposition system was used to prepare samples for analysis of heavy metals. A mixture of 70% nitric acid and 30% hydrogen peroxide (10:1 vol, 180°C, 25 min) was used as an oxidizing agent for sample mineralization. TLC analysis was performed on ALUGRAM Xtra SIL G/UV₂₅₄ aluminum plates (Macherey-Nagel). The plates were visualized with a 254 nm ultraviolet lamp. The completeness of the reactions and the evaluation of the chromatographic purity of the intermediates were monitored using an Alliance HPLC system (Waters) with a photodiode detector array (PDA).

### Analytical conditions

### Method A

Instrument: Waters Alliance HPLC; Column XBridge C18, 3.5 µm, 4.6 mm × 150 mm; Eluent A: MeCN; Eluent B: H₂O + 0.15% vol. 50% H₃PO₄; Eluent ratio A:B = 50:50; Isocratic elution mode; Flow rate 1.50 ml/min; Temperature 40°C; Injection volume 10 µl; Detection wavelength: 190-350 nm.

### Method B

Instrument: Waters Alliance HPLC; Column XBridge C18, 3.5 µm, 4.6 mm × 150 mm; Eluent A: MeCN; Eluent B: H₂O + 0.15% vol. 50% H₃PO₄; Eluent ratio A:B = 30:70; Isocratic elution mode; Flow rate 1.00 ml/min; Temperature 40°C; Injection volume 10 µl; Detection wavelength: 190-350 nm.

### Method C

Instrument: Waters Alliance HPLC; Column XBridge C18, 3.5 µm, 4.6 mm × 150 mm; Eluent A: MeCN; Eluent B: H₂O + 0.15% vol. 50% H₃PO₄; Gradient: 0-1.20 min 30% A, 1.20-12.00 min 30-90% A, 12.00-15.00 min 90% A, 15.01-18.00 min 90-30% A; Flow rate 1.20 ml/min; Temperature 40°C; Injection volume 10 µl; Detection wavelength: 190-350 nm.

### Experimental

### 2,3-dimethylbromobenzene (11)

### (Modified procedure from J. Chem. Soc. 1940, 16-18)

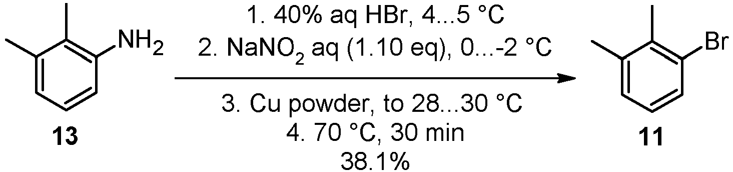

3,747.5 g (22.23 mol; 4.0 eq) of 48% hydrobromic acid and 750 ml of deionized water are charged into a 10 L glass reactor with a thermostatically controlled jacket, equipped with a mechanical stirrer, pre-cooled down to -10°C. The solution is cooled down to 4 ... 5°C, and 673.45 g (5.55 mol; 1.0 eq) of 2,3-dimethylaniline **13** is added with stirring (250-300 rpm) from a dropping funnel within 45 minutes, at such a rate that the temperature of the reaction mixture was kept in the range of 4... 5°C. The resulting suspension of beige crystalline 2,3-dimethylaniline hydrobromide is cooled down to -1 ... -2°C, and a solution of 421.8 g (6.12 mol; 1.10 eq) of sodium nitrite in 680 ml of deionized water is added from a dropping funnel within 2 hours, with such a rate that the temperature of the reaction mixture would be kept in the range of 0 ... -2°C (the temperature of the coolant in the reactor jacket being -15°C). After adding the sodium nitrite solution, the temperature of the reaction mixture is raised up to 0 °C, and the yellow-brown solution of 2,3-dimethylphenyldiazonium bromide **12** is stirred for 15 min to complete the diazotization process. Further, the stirrer speed is increased (up to 450-500 rpm), a suspension of 100 g of freshly deposited copper powder in 350 ml of deionized water is introduced into the reactor, and the coolant temperature in the reactor jacket is increased up to 30°C. When the reaction mixture is heated up to 28...30°C, the exothermic decomposition process of the diazonium salt begins with a spontaneous increase in the temperature of the reaction mixture up to 60°C within 4-5 min. After the spontaneous temperature rise ceases, heating and circulation of the coolant in the reactor jacket are started, and the violet-black reaction mixture is heated at 70°C for 30 min. The reaction mixture is cooled down to room temperature and extracted with 2×1,000 ml of light petroleum ether (40-70). The lower aqueous layer is drained through the bottom outlet, and the dark organic phase is successively washed in the reactor with 4 L of deionized water (10 min), and 4×4 L of 2.5% sodium hydroxide solution (10 min each) to remove 2,3-dimethylphenol. The organic phase is separated and concentrated in vacuum (<40°C). The resulting orange-red oil is steam distilled from a solution containing 50 g of sodium hydroxide in 1 L of deionized water, collecting 3.5 L of distillate. The transparent lower layer of light orange color is separated and intensively stirred for 40 min with 100 ml of concentrated sulfuric acid on a magnetic stirrer. The lower dark red sulfuric acid layer is separated in a separating funnel, and the upper light yellow organic layer is washed with 2×600 ml of saturated sodium bicarbonate solution. The aqueous phase is extracted with 3×100 ml of methylene chloride. The combined organic phase is dried with anhydrous sodium sulfate, filtered and concentrated in vacuum. The resulting 2,3-dimethylbromobenzene was fractionally distilled in vacuo using a 300 mm vacuum-jacketed Vigreux column to collect a fraction with bp 76.2-76.5°C/6 mmHg.

**Yield 391.8 g (38.1%).** Colorless liquid with a characteristic xylene-like odor. ¹H NMR (400 MHz, CDCl₃) δ 7.43 (d, 1H), 7.11 (d, 1H), 6.97 (t, 1H), 2.40 (s, 3H), 2.36 (s, 3H); ¹³C (100 MHz, CDCl₃) δ 138.61, 136.24, 130.34, 128.97, 126.88, 125.67, 21.39, 19.51; tR 11.33 min (HPLC Area 99.7%, method A).

### 3-bromo-2-butanone (15)

### (modified procedure from Rec. Trav. Chim. Pays-Bas 1946, 65, 691).

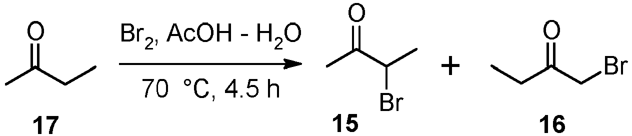

1,200 ml of deionized water, 300 ml of glacial acetic acid and 360.5 g (447.8 ml; 5.0 mol) of methyl ethyl ketone **17** are charged into a 3 L glass reactor with a thermostatically controlled jacket, equipped with a mechanical stirrer with a hermetic seal, a dropping funnel with a tube for pressure equalization and a reflux condenser. 800.0 g (257.9 ml; 5.0 mol; 1.0 eq) of bromine are charged into the dropping funnel. The solution in the reactor is heated up to 70°C, and bromine is added dropwise over 4 hours, at such a rate that the yellow color appearing at the beginning has time to disappear with the addition of subsequent bromine portions. During bromine addition, an emulsion is formed, and then the reaction mixture separates into 2 phases. After all of the bromine has been added, the reaction mixture is stirred at 70°C for an additional 30 min, then cooled down to room temperature and diluted with 1 L of cold water (2°C). The lower light yellow layer is separated through the bottom outlet of the reactor, and washed with 3 ×400 ml of a saturated sodium bicarbonate solution (on a magnetic stirrer, in a loosely closed 1,000 ml conical flask), until the evolution of carbon dioxide ceases, and then with 400 ml of deionized water. The resulting mixture of regioisomeric bromobuta-nones **15/16** (546.1 g) is dried with anhydrous granular calcium chloride, filtered and separated by vacuum distillation on a distillation column (filled with Raschig rings) 800 mm high with an evacuated jacket. The first portion of the distillate (about 10 ml) with bp 58-60°C/38 mmHg is separated, then the main fraction of 3-bromo-2-butanone **15** with bp 61-63°C/38 mmHg is collected, then the intermediate fraction with bp 64-77°C/38 mmHg is collected, and finally the fraction of 1-bromo-2-butanone **16** with bp 77-78°C/38 mmHg is collected. Running vacuum distillation at too low a rate would cause the distillate to turn yellow.

### 3-bromo-2-butanone (15)

**Yield 279.74 g (37.0%).** Light yellow liquid. ¹H NMR (400 MHz, CDCl₃) δ 4.37 (q, 1H), 2.34 (s, 3H), 1.71 (d, 3H); ¹³C (100 MHz, CDCl₃) δ 202.11, 48.28, 26.04, 20.20. Content of regioisomeric 1-bromobutan-2-one **16** <5% (NMR). tR 5.35 min (HPLC Area 96.9%, method B).

### 1-bromo-2-butanone (16)

**Yield 48.15 g (6.4%).** Yellow liquid. ¹H NMR (400 MHz, CDCl₃) δ 3.87 (s, 2H), 2.65 (q, 2H), 1.09 (t, 3H); ¹³C (100 MHz, CDCl₃) δ 209.92, 52.65, 29.13, 9.35; tR 4.64 min (HPLC Area 88.9%, method B).

### 2-(1-bromoethyl)-2-methyl-1,3-dioxolane (14)

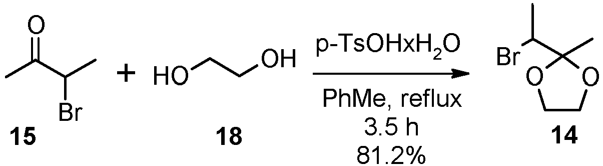

259.12 g (1.71 mol) of freshly distilled 3-bromo-2-butanone **15,** 117.2 g (1.88 mol; 1.10 eq) of ethylene glycol, 1.58 g (8.30 mmol; 0.0048 eq) of *p-*toluenesulfonic acid monohydrate and 800 ml of toluene are charged into a 2 L round-bottomed flask. The flask is equipped with a 50 ml Dean-Stark trap under reflux, and the reaction mixture is refluxed with stirring on a magnetic stirrer until no more water droplets separate in the receiver (31 ml; 3.5 hours). As the reaction proceeds, the bottom layer of ethylene glycol disappears. The clear colorless solution is cooled down to room temperature, washed with 500 ml of 2% sodium hydrogen carbonate solution, and 2×150 ml of deionized water. The organic layer is separated, dried over anhydrous sodium sulfate, filtered and concentrated on a rotary evaporator (35°C/10 mmHg), focusing on the distillation volume (760-780 ml). The product is distilled in vacuum, collecting the fraction with bp 43.5-44.5°C/0.016 mmHg.

**Yield 271.93 g (81.2%).** Colorless liquid with no lachrymatory action. ¹H NMR (400 MHz, CDCl₃) δ 4.05 (q, 1H), 3.99 (m, 4H), 1.67 (d, 3H), 1.45 (s, 3H); ¹³C (100 MHz, CDCl₃) δ 109.96, 64.74, 53.22, 21.22, 20.65; tR 8.11 min (HPLC Area 88.2%, method B).

### 3-(2,3-dimethylphenyl)butan-2-one (7)

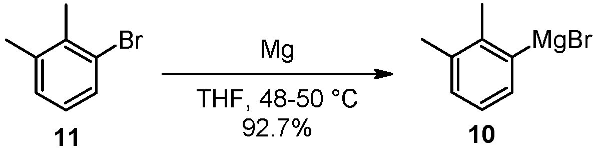

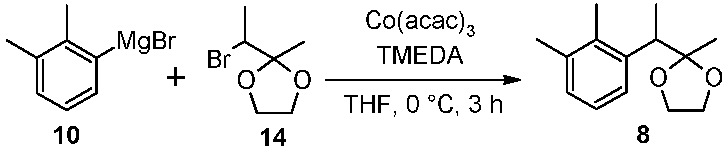

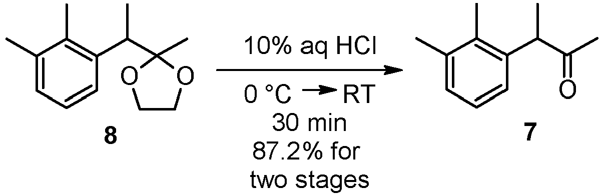

33.90 g (1.39 mol; 1.05 eq) of magnesium turnings were placed in a 2 L two-neck round-bottom flask, which was preliminarily dried overnight in a drying box at 120 °C and equipped with a 1 L dropping funnel with a pressure equalizing arm and a glass-enclosed thermocouple. About 0.8 g of iodine is added to the flask, and the flask is heated with hot air until the iodine starts to sublime. After cooling the flask down to room temperature, the magnesium is covered with 147 ml of dry THF and a magnetic anchor is placed into the flask. A solution of 245.67 g (1.32 mol; 1.0 eq) of 2,3-dimethylbromobenzene **11** in 1,000 ml of dry THF is loaded into the dropping funnel. About 40-50 ml of the solution is added to the magnesium turnings from the dropping funnel in one portion. The reaction starts immediately after adding the first portion of the solution from the dropping funnel, the iodine color disappears, and the temperature rises rapidly (<1 min) up to 48°C. After the reaction mixture temperature stops rising, adding the solution from the dropping funnel is immediately started with active stirring (1300-1500 rpm), at such a rate that the temperature of the reaction mixture is maintained in the range of 47-49°C without external heating or cooling (about 5 h). After all 2,3-dimethylbromobenzene was added, the dark reaction solution is refluxed at 70-75°C for 45 min to complete the reaction. The solution is cooled in an argon atmosphere. The concentration of 2,3-dimethylphenylmagnesium bromide **10** in the resulting solution, determined by the method of Paul Knochel, is 0.99 mol/1. **The yield of 2,3-dimethylphenylmagnesium bromide is 92.7%.** The resulting solution of compound 10 is directly used in the next step without further preparation.

A 5 L glass reactor with a temperature-controlled jacket, equipped with a mechanical stirrer with a hermetic seal, a thermocouple, and a stopcock for introducing an inert gas is evacuated (<0.1 mmHg) and dried at 90°C for 2 hours. The reactor is cooled down to room temperature and filled with dry argon (3 pump/fill cycles). 41.93 g (10 mol%; 117.7 mmol) of cobalt (III) acetylacetonate, 520 ml of dry THF, 17.65 ml (13.67 g; 10 mol%; 117.7 mmol) of TMEDA, and 229.67 g (1.177 mmol; 1.0 eq) of 2-(1-bromoethyl)-2-methyl-1,3-dioxolane **14** are consecutively added to the dried reactor in a countercurrent of argon. The suspension is cooled down to 0 ... -1°C, and a solution of 2,3-dimethylphenylmagnesium bromide **10,** prepared in the previous stage, is added to the reactor using a peristaltic pump at a rate of 6.9-7 ml/min under stirring (300-400 rpm) for 180 min, carefully maintaining the internal temperature in the range from 0 to -1°C (the temperature of the coolant in the reactor jacket, providing good control over the reaction, is from -10 to -12°C). As the Grignard reagent solution is added, the color of the reaction mixture changes from green to light blue-blue, and a bulky precipitate of magnesium bromide crystallizes out of the solution. After adding all 2,3-dimethylphenylmagnesium bromide (1.05 eq), the temperature of the reaction mixture is raised up to 20°C, and stirring was continued for 1 hour. 1,460 ml of 9% hydrochloric acid were slowly (over 2-3 min) added to the reaction mixture under intense stirring (400 rpm), and stirring was continued for 40 min. During the addition of hydrochloric acid, the color of the reaction mixture rapidly changed from light blue-blue to green, and then orange. The entire crystalline precipitate of magnesium bromide goes into solution. HPLC analysis of an aliquot of the reaction mixture, taken after 30 min of stirring, shows 100% conversion of intermediate ketal **8** to ketone **7.** The reaction mixture consisting of two phases (upper, light yellow organic and lower pink-red aqueous) is diluted with 500 ml of n-heptane, the phases are stirred for 15 min, and the lower aqueous layer is drained through the bottom outlet of the reactor. The organic phase is washed successively with 2×500 ml of deionized water and 500 ml of saturated sodium hydrogen carbonate solution. The organic phase is dried with sodium sulfate, filtered and concentrated in vacuum (45°C). The obtained orange-yellow oil (224.0 g) is diluted with 240 ml of methanol, and 100 mg of 2,2',3,3'-tetramethylbiphenyl **9** is introduced into the solution as a seed. The solution is kept in a freezer (-18°C) during a day. The suspension of crystals of precipitated 2,2',3,3'-tetramethylbiphenyl **9** is filtered on a Schott funnel and washed with 2×50 ml of cold methanol. The filtrate is concentrated in vacuum; the residue is diluted with 300 ml of hexane and successively washed to remove ethylene glycol with 1 L of deionized water (10 min), 1 L of 1% aqueous Na₂EDTA solution (2 hours), 1 L of saturated aqueous sodium chloride solution (10 min). The completeness of the removal of ethylene glycol is monitored using 1H NMR by the disappearance of the signal of methylene groups (3.76 ppm, s, CDCl₃).

The organic phase was separated, dried with anhydrous sodium sulfate, filtered, and concentrated in vacuo. The product was vacuum-distilled using a 300 mm vacuum-jacketed Vigreux column. The distillate was collected in a Perkin receiver. The fraction boiling up to 68-69 °C/0.024 mm Hg was discarded, and the main product fraction with bp 69-72 °C/0.024 mm Hg was collected.

The resulting amber-colored 3-(2,3-dimethylphenyl)butan-2-one 7 is diluted with 250 ml of hexane and stirred for 1.5 hours with 10 g of silica gel (40-63 µm, dried at 120°C for 20 hours). The orange-colored silica gel is filtered on a Schott funnel, washed with 3×20 ml of hexane, and the filtrate is concentrated in vacuum on a rotary evaporator. The residue is kept under vacuum (<0.1 mmHg, RT, 30 min) to remove hexane traces. **Yield 161.22 g (77.7%).** Another **19.63 g** of the product can be isolated by extraction of the combined aqueous phase (4 L) obtained after quenching the reaction mixture with 2×200 ml of heptane, followed by purification as described above.

### 3-(2,3-dimethylphenyl)butan-2-one (7)

**Total yield 180.85 g (87.2%).** Light yellow liquid.¹H NMR (400 MHz, CDCl₃) δ 7.08 (d, 2H), 6.91 (t, 1H), 3.97 (q, 1H), 2.32 (s, 3H), 2.27 (s, 3H), 2.01 (s, 3H), 1.35 (d, 3H); ¹³C (100 MHz, CDCl₃) δ 209.63, 139.22, 137.61, 134.50, 128.90, 126.18, 124.99, 50.58, 28.44, 21.14, 16.93, 15.33; tR 4.88 min (HPLC Area 99.2%, method A).

### 2,2',3,3'-tetramethylbiphenyl (9)

**Yield 10.08 g (7.2%).** White crystalline substance. ¹H NMR (400 MHz, CDCl₃) δ 7.14 (m, 4H), 6.96 (d, 2H), 2.34 (s, 6H), 1.96 (s, 6H); ¹³C (100 MHz, CDCl₃) δ 142.48, 136.79, 134.67, 128.63, 127.41, 125.19, 20.68, 16.55; tR 42.3 min (HPLC Area 98.8%, method A).

### 1-bromo-3-(2,3-dimethylphenyl)butan-2-one (5)

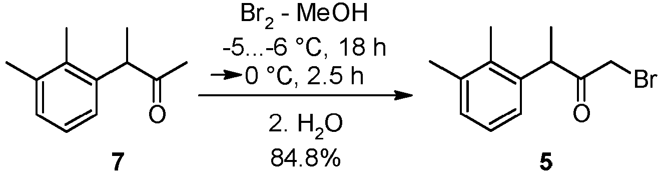

A 5 L glass reactor with a temperature-controlled jacket, equipped with a mechanical stirrer with a hermetic seal, a thermocouple, and a stopcock for introducing an inert gas is evacuated (<0.1 mmHg) and dried at 90 °C for 2 hours. The reactor is cooled down to room temperature and filled with dry argon (3 pump/fill cycles). 114.57 g (0.65 mol; 1.0 eq) of 3-(2,3-dimethylphenyl)butan-2-one 7 in 3,420 ml of anhydrous methanol is charged into the dried reactor in a countercurrent of argon. The solution is cooled down to -10°C, and a solution of 124.65 g (0.78 mol; 1.20 eq) of bromine in 592 ml of methanol is poured (in 30-35 s) into the reactor with stirring (250 rpm) (temperature rises up to -9°C). The temperature of the reaction mixture is smoothly raised up to -5...-6°C, and stirring is continued at this temperature for 18 hours, while monitoring the reaction progress by HPLC (method A). Next, the temperature of the reaction mixture (light orange) is gradually increased up to 0°C, an additional 5.0 g (0.03 mol) of bromine is added, and stirring is continued for 2.5-3 hours to complete the reaction, monitoring the conversion of the starting ketone using HPLC (method A). After full completion of the reaction (>97% conversion of the starting ketone), the clear solution is poured into a 10 L stirred reactor containing 7,200 ml of deionized water pre-cooled down to 0.5°C. The resulting white emulsion is stirred for 30 min and extracted with 4×300 ml of methylene chloride. The organic extract is separated through the bottom outlet of the reactor, combined and washed with 1 L of saturated aqueous sodium hydrogen carbonate solution (300 rpm, 30 min), 1 L of deionized water (300 rpm, 30 min), and dried with anhydrous sodium sulfate. The solution is filtered and the solvent is removed in vacuum (<30°C). The residue is used directly in the next step without further purification.

**Yield 171.57 g (84.8%).** Light yellow transparent oil with no lachrymatory action. ¹H NMR (400 MHz, CDCl₃) δ 7.08 (d, 2H), 6.85 (t, 1H), 4.39 (q, 1H), 3.79 (dd, 2H), 2.33 (s, 3H), 2.31 (s, 3H), 1.40 (d, 3H); ¹³C (100 MHz, CDCl₃) δ 202.70, 138.10, 138.01, 134.54, 129.40, 126.43, 124.95, 46.75, 33.25, 21.04, 17.29, 15.34; tR 8.16 min (HPLC Area 82.0%, method A).

### 3-bromo-3-(2,3-dimethylphenyl)butan-2-one (20)

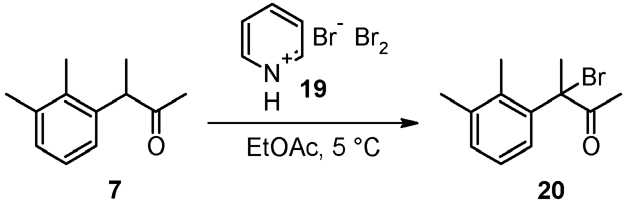

8.0 g (45.4 mmol; 1.0 eq) of 3-(2,3-dimethylphenyl)butan-2-one 7 in 80 ml of ethyl acetate are loaded into a 100 ml glass reactor with a thermostatically controlled jacket, equipped with a mechanical stirrer with wide blades and a hermetic seal, a valve for inert gas introduction. The solution is cooled down to 5°C, a weak flow of argon (100 ml/min) is started, and 15.25 g (47.6 mmol; 1.05 eq) of pyridine hydrobromide perbromide **19** are sprinkled through a plastic funnel in portions of approx. 500-600 mg, at intervals of approx. 1 min with intense stirring (300 rpm). The color of the reagent instantly disappears, and a crystalline white flocculent precipitate of pyridinium hydrobromide begins to fall out of the solution. It takes about 30 min to add the entire reagent. The reaction mixture is stirred at 5°C for 40 min and then an additional 1.5 g of pyridinium perbromide bromide is added (the total amount of the brominating reagent being 16.75 g). The reaction mixture is stirred for 10 min and diluted with 100 ml of deionized water. Stirring is continued for 5 min to dissolve the precipitate of pyridinium hydrobromide, the lower aqueous phase is drained, and the washing of the organic phase is repeated with another 100 ml of deionized water, actively mixing the layers for 5 min. The aqueous phase is drained through the bottom outlet of the reactor, and washing is repeated with 75 ml of saturated sodium hydrogen carbonate solution. The layers are stirred vigorously for 40 min at room temperature, separated, and the light yellow ethyl acetate layer is finally washed with 50 ml of deionized water. The aqueous phase is extracted with 20 ml of ethyl acetate; the combined organic extract is dried over anhydrous sodium sulfate, filtered and concentrated in vacuum at 40°C. The residue is kept under vacuum to remove ethyl acetate traces.

**Yield 12.0 g (72.5%).** Light orange oil crystallizing in a freezer (-18°C) into yellow needles. ¹H NMR (400 MHz, CDCl₃) δ 7.69 (m, 2H), 7.16 (m, 1H), 2.27 (s, 3H), 2.24 (s, 3H), 2.22 (s, 3H), 2.09 (s, 3H); ¹³C (100 MHz, CDCl₃) δ 203.10, 138.75, 138.57, 133.86, 130.64, 126.19, 126.11, 73.48, 29.79, 25.29, 20.84, 17.16; tR 7.58 min

### (HPLC Area 70%, method A)

### 3-(2,3-dimethylphenyl)-2-oxobutyl acetate 4 (Route A)

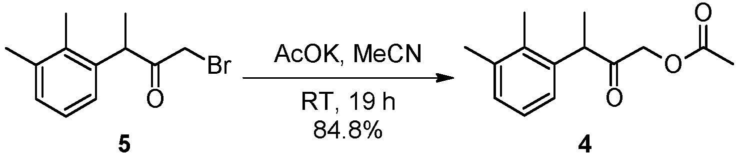

191.4 g (1.95 mol; 3.5 eq) of potassium acetate and 1,000 ml of acetonitrile are charged into a 2 L round bottom flask. A solution of 171.57 g of crude 1-bromo-3-(2,3-dimethylphenyl)butan-2-one (the calculated content of pure compound **5** is 140.68 g; 0.55 mol) in 850 ml of acetonitrile is added to the flask in one portion, and the suspension is stirred on a magnetic stirrer (1,500 rpm) at room temperature (21°C), monitoring the progress of the reaction with HPLC (method A). After completion of the reaction (19 hours, >99% conversion), the crystalline precipitate of potassium bromide and excess potassium acetate are filtered on a Schott funnel and washed on the filter with 2×200 ml of acetonitrile. The clear light yellow filtrate is concentrated in vacuum (<40°C), and the residue is diluted with 300 ml MTBE. The organic phase is washed with 2×500 ml of deionized water, the aqueous phase is further extracted with 2x50 ml of MTBE and the combined organic phase is dried over anhydrous sodium sulfate. The solution is filtered, and the solvent is removed in vacuum (<35°C). The residue is used directly in the next step without further purification.

**Yield 158.07 g (84.8%).** Yellow oil. tR 4.54 min (HPLC Area 69.3%, method A).

A sample of compound **4** was purified for NMR analysis by flash chromatography (silica gel 60, hexane-ethyl acetate 9:1, Rf 0.16). Purified compound **4** is a white waxy mass. ¹H NMR (400 MHz, CDCl₃) δ 7.08 (d, 2H), 6.91 (t, 1H), 4.53 (dd, 2H), 4.04 (q, 1H), 2.31 (s, 3H), 2.27 (s, 3H), 2.09 (s, 3H), 1.39 (d, 3H); ¹³C (100 MHz, CDCl₃) δ 204.59, 170.26, 137.86, 137.80, 134.34, 129.27, 126.36, 125.13, 67.08, 46.93, 21.10, 20.48, 16.73, 15.30.

### Medetomidine hydrochloride 1 (Route A)

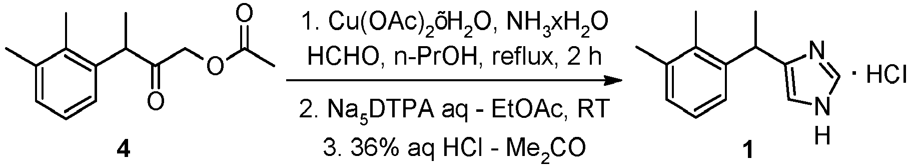

A 10 L glass jacketed reactor equipped with a mechanical stirrer, a thermocouple and an efficient reflux condenser is charged with a solution of 158.07 g of crude 3-(2,3-dimethylphenyl)-2-oxobutyl acetate (the calculated amount of pure compound **4** being 109.54 g; 0.467 mol) in 1,700 ml of *n*-propanol. A solution of 268.72 g (1.34 mol) of copper (II) acetate monohydrate in 2,529 ml (2293.9 g; 33.6 mol) of 25% aqueous ammonia solution is added to the reactor with stirring (160 rpm) in one portion. The homogeneous dark blue solution is stirred for 3 min and 490 ml (6.64 mol) of 37% formalin are added to the reactor in one portion. The reaction mixture is heated up to boiling (heating temperature 100°C) with stirring (160 rpm) for 2 hours. Approx. 10-15 min after the start of heating, the precipitation of crystalline copper (I) complex of medetomidine 3 begins. After 2 hours of heating, the reaction suspension is cooled down to 16-18°C for 1 hour, and stirring is continued for another 30 min at 16-18°C. The precipitate is filtered on a Schott funnel and washed successively with 1 L of 25% aqueous *n*-propanol, 1 L of deionized water and 2×250 ml of acetone. The resulting dark yellow fine crystalline powder is dried in vacuum (6 mmHg) at room temperature for 12 hours. **The yield of the copper (I) complex of medetomidine was 99.68 g (58.1% in 3 stages).**

A 10 L glass reactor equipped with a mechanical stirrer is charged with 2,850 g (0.566 mol; 1.50 eq) of a 10% aqueous solution of diethylenetriaminepentaacetic acid pentasodium salt (NasDTPA) and 99.68 g (0.377 mol) of the resulting copper (I) complex of medetomidine **3,** suspended in 2 L of ethyl acetate. The phases are stirred (300-350 rpm) at room temperature (23°C) for 24 hours until all solids are completely dissolved. The dark ethyl acetate layer is separated from the intense blue colored aqueous phase. The aqueous phase is extracted with 2×200 ml of ethyl acetate. The combined organic phase containing medetomidine base is transferred to a 5 L stirred glass reactor and washed successively with 2×500 ml of 0.5% aqueous NasDTPA solution (30 min each) and 500 ml of saturated aqueous sodium chloride solution (30 min). Medetomidine is removed from the ethyl acetate phase by sequential extraction with 500 ml and 2×350 ml of 10% aqueous acetic acid solution (stirring at 300-400 rpm, 10 min for each portion). The medetomidine-depleted ethyl acetate layer is concentrated in vacuum to -200 ml, and additional medetomidine is extracted with 2×300 ml of 10% aqueous acetic acid solution. The combined aqueous extract (1,800 ml) containing medetomidine acetate is transferred to a 5 L stirred glass reactor and extracted successively with 3×200 ml of MTBE to extract minor impurities. At the same time, the aqueous phase becomes brighter. The dark organic extract (MTBE) is washed once with 100 ml of deionized water, the aqueous phase is combined with the extract containing medetomidine acetate, and the organic phase is discarded. The combined light yellow aqueous phase (1,900 ml) is transferred to a 5 L glass jacketed reactor equipped with a mechanical stirrer, cooled down to 8-10°C and neutralized with 350 ml of 25% aqueous ammonia solution under stirring. The separated medetomidine base **2** is extracted with 3×250 ml of ethyl acetate, the organic layer is washed once with 250 ml of a saturated aqueous solution of sodium chloride, dried over anhydrous sodium sulfate, filtered and concentrated in vacuum (carefully because foaming is possible). The product is a honey-like substance, amber in color, foaming in vacuum and solidifying in the form of fragile pearly foam. **The yield of crude medetomidine base is 65.74 g.**

The base of medetomidine **2** is dissolved in 250 ml of dry acetone, directly in the flask which the concentration was carried out in. The clear dark solution is cooled down to -10°C, and 1.0 g of crystalline medetomidine hydrochloride is added as a seed. The cooled solution under stirring (500-600 rpm) is acidified dropwise with 28.0 ml of 36% hydrochloric acid to pH 2-3. Crystallization of a well-formed precipitate of medetomidine hydrochloride **1** begins. The crystallization process is allowed to complete at -18 ... -20°C for 23 hours, the product is filtered on a Schott funnel, washed with 3×100 ml of cold (2-3°C) dry acetone and dried in vacuum at 40°C for 12 hours.

**The yield of medetomidine hydrochloride is 50.25 g (56.2% in 2 stages).** Off-white fine crystalline powder (HPLC Area 95.3%, method C). Residual copper content 3.7 ppm (AAS).

For purification, the product is crystallized from acetone containing 3.85% (vol.) water (approx. 9.5 ml of solvent per 1 g of medetomidine hydrochloride). Yield 90% (one purification step, HPLC Area 98.5%, method C). After crystallization twice, the yield is 78-82% (HPLC area 99.5%, method C). Residual copper content after recrystallization <1 ppm (AAS).

White fine crystalline powder. ¹H NMR (400 MHz, D₆-DMSO) δ 9.02 (s, 1H), 7.48 (s, 1H), 7.04 (m, 2H), 6.83 (m, 1H), 4.51 (q, 1H), 2.26 (s, 3H), 2.24 (s, 3H), 1.51 (d, 3H); ¹³C (100 MHz, D₆-DMSO) δ 141.04, 137.36, 136.71, 134.07, 133.78, 128.30, 125.69, 124.05, 115.80, 31.88, 20.61, 20.31, 14.51; tR 3.11 min (HPLC, method C).

### Sodium diformylamide 21 (Route B)

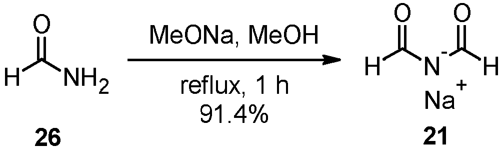

28.42 g (526 mmol) of powdered sodium methoxide and 100 ml of dry methanol are charged into a 500 ml round-bottomed flask filled with argon (be careful, warm up!) and stirred until complete dissolution. 47.39 g (1.052 mol; 2.0 eq) of formamide is added dropwise to the resulting solution over 2-3 min. The solution is heated at reflux temperature for 1 hour under stirring on a magnetic stirrer. Then, the solution is cooled slightly, and the reflux condenser is changed to a distillation bridge with a descending condenser and a Claisen nozzle equipped with a thermometer and a dropping funnel containing 132 ml of toluene. The methanol is distilled off by measuring the volume of distillate with a graduated cylinder, and after collecting about 80 ml of distillate, the addition of toluene from the dropping funnel is started, at such a rate as to maintain a constant volume of solution in the distilling flask. The distillate is collected until the vapor temperature in the Claisen nozzle reaches 110°C (the total volume of distillate is about 150 ml). The white suspension is cooled down to room temperature and filtered on a Schott funnel. The product is washed with 80 ml THF and dried in vacuum (0.05 mmHg) at room temperature for 1 hour. The resulting product is used directly in the next step without further purification.

### Yield 45.68 g (91.4%). White fine crystalline powder.

### N-[3-(2,3-dimethylphenyl)-2-oxobutyl]-N-formylformamide 22 (Route B)

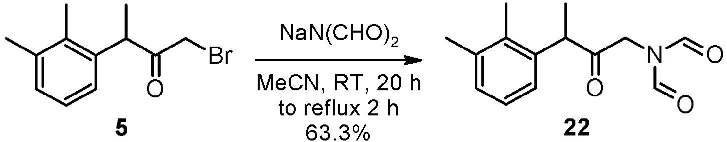

A 2 L two-necked round-bottomed flask filled with argon (3 pump/fill cycles) is charged with 38.46 g (404.7 mmol; 1.4 eq) of sodium diformylamide **21** and 400 ml of acetonitrile (water content <0.05%). A solution of 87.50 g of crude 3-(2,3-dimethylphenyl)-1-bromobutan-2-one **5** (chromatographic purity 82%; 281.2 mmol) in 400 ml of acetonitrile is added to the resulting suspension in one portion under stirring on a magnetic stirrer. The reaction mixture is magnetically stirred under argon atmosphere at room temperature for 20 hours, after which the mixture was refluxed for 2 hours to achieve >99% conversion of starting compound **5** (HPLC, method A). The hot dark yellow solution is filtered on a Schott funnel, and the precipitate of inorganic salts is washed with 50 ml of acetonitrile. The clear filtrate is concentrated in vacuum (40°C), and the dark yellow oil obtained is washed with 350 ml of deionized water under stirring on a magnetic stirrer for 10 min. The product is extracted with 300 ml of methylene chloride, the organic phase is separated and washed with 350 ml of deionized water. The aqueous phase is re-extracted with 30 ml of methylene chloride and the combined organic phase is concentrated in vacuum. The resulting yellow viscous oil is diluted with 250 ml of hexane, directly in the same flask which the concentration was carried out in, and the flask is heated in an oil bath (90°C) under reflux with stirring on a magnetic stirrer for 30 min. The mixture is cooled, the upper hexane layer is carefully drained and discarded, and the remaining oil is washed again with 100 ml of hexane. The hexane layer is discarded and the residual hexane is removed from the product in vacuum. The residue is used directly in the next step without further purification.

**Yield 51.24 g (63.3%).** Yellow viscous oil. ¹H NMR (400 MHz, CDCl₃) δ 8.88 (s, 2H), 7.10 (m, 2H), 6.95 (m, 1H), 4.30 (dd, 2H), 4.03 (q, 1H), 2.33 (s, 3H), 2.26 (s, 3H), 1.42 (d, 3H); ¹³C (100 MHz, CDCl₃) δ 209.80, 163.27, 138.01, 137.73, 134.60, 129.50, 126.53, 125.59, 48.58, 46.11, 21.10, 16.79, 15.39; tR 3.15 min (HPLC Area 85.9 %, method A).

### 1-amino-3-(2,3-dimethylphenyl)butan-2-one hydrochloride 23 (Route B)

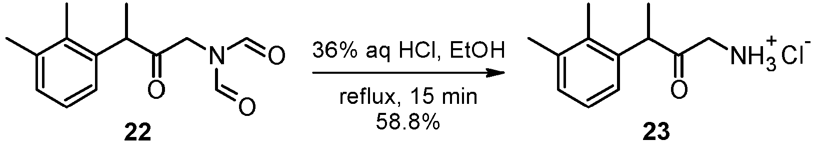

47.26 g of crude N-[3-(2,3-dimethylphenyl)-2-oxobutyl]-N-formylformamide **22** (chromatographic purity 85.9%; 164.1 mmol) obtained in the previous step is dissolved in 600 ml of 99% ethanol, and 65.0 ml of 36% (764 mmol; 4.6 eq) hydrochloric acid is added to the obtained clear yellow solution in one portion. The solution is heated up to boiling under argon while stirring on a magnetic stirrer. HPLC analysis of an aliquot of the reaction mixture taken after 15 min shows >99% conversion of starting compound **22** to aminoketone **23** (HPLC, method A). The violet-red solution is concentrated in vacuum, and the crystalline residue is dried at 40°C/2 mmHg for 1 hour (on a rotary evaporator). The residue is suspended in 350 ml of diethyl ether; the suspension of crystals is stirred on a magnetic stirrer for 10-15 minutes, filtered on a Schott funnel and washed with 3x100 ml of acetone. The resulting aminoketone hydrochloride **23** (24.91 g) is recrystallized from a mixture of 315 ml of acetone and 30 ml of deionized water, and dried in vacuum (6 mmHg) at room temperature for 12 hours.

**Yield 22.0 g (58.8%).** Colorless shiny needles. ¹H NMR (400 MHz, D₆-DMSO) δ 8.40 (s, 3H), 7.08 (m, 2H), 6.88 (m, 1H), 4.25 (q, 1H), 3.73 (dd, 2H), 2.26 (s, 3H), 2.21 (s, 3H), 1.29 (d, 3H); ¹³C (100 MHz, D₆-DMSO) δ 204.41, 137.95, 137.22, 134.53, 128.83, 125.92, 125.00, 46.37, 45.45, 20.66, 16.58, 15.01; tR 3.10 min (HPLC Area 97.3%, method B).

### 4-[1-(2,3-dimethylphenyl)ethyl]-1,3-dihydro-2H-imidazole-2-thione 24 (Route B)

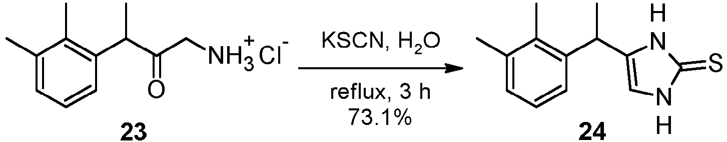

12.63 g (55.4 mmol) of 1-amino-3-(2,3-dimethylphenyl)butan-2-one hydrochloride **23** and 70 ml of deionized water are placed into a 250 ml round-bottomed flask. The suspension of crystals is heated in an oil bath up to 50-55°C and stirred for 5 min. 26.95 g (277.3 mmol; 5.0 eq) of potassium thiocyanate is added to the suspension with stirring on a magnetic stirrer in one portion, the flask is equipped with a reflux condenser, and the reaction mixture is heated at 125-130°C (bath temperature). After approx. 10 min of heating, all solids dissolve to form a clear yellowish solution, which, on further heating (approx. 30-40 min), the product begins to precipitate from. After completion of the reaction (total heating time 3 hours 20 min), the crystal suspension is cooled down to room temperature and then down to 0°C. The crystals are filtered on a Schott funnel, washed with 3×100 ml of deionized water, and a minimum amount of cold 96% ethanol. The product is dried under vacuum (6 mmHg) at room temperature for 12 hours.

**Yield 9.42 g (73.1%).** Light yellow fine-crystalline shiny powder, odorless. ¹H NMR (400 MHz, D₆-DMSO) δ 11.83 (s, 1H), 11.70 (s, 1H), 7.02 (m, 2H), 6.92 (m, 1H), 6.50 (s, 1H), 4.15 (q, 1H), 2.24 (s, 3H), 2.20 (s, 3H), 1.38 (d, 3H); ¹³C (100 MHz, D₆-DMSO) δ 141.71, 136.26, 133.71, 133.58, 133.44, 127.89, 125.36, 123.93, 111.40, 31.79, 20.61, 19.92, 14.39; tR 9.49 min (HPLC Area 98.9%, method B).

### Medetomidine 2 (Route B)

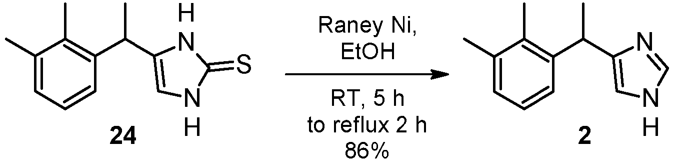

7.90 g (34.0 mmol) of 4-[1-(2,3-dimethylphenyl)ethyl]-1,3-dihydro-2H-imidazol-2-thione **24** and 50 ml of 99 % ethanol are placed into a 500 mL Schlenk flask filled with argon. In a countercurrent of argon, 35 ml of a freshly prepared suspension of Raney nickel W-4 in 99% ethanol (nickel content in the suspension being 0.28 g/ml; 9.8 g; 167 mmol; 4.9 eq) is transferred into the flask, and the contents are stirred for 1.5 hours at room temperature with a magnetic stirrer. After 1.5 hours of stirring, a second portion (35 ml; 4.9 eq) of the suspension of Raney nickel W-4 in 99% ethanol is added and stirring is continued at room temperature for 3.5 hours. After the indicated time, a third portion (10 ml; 2.8 g; 47.7 mmol; 1.4 eq) of the suspension of Raney nickel W-4 in 99% ethanol is added, and the reaction mixture is heated under stirring in an inert atmosphere at 95°C for 2 hours, for full completion of the reaction (conversion of the starting compound **24** ≥99.5%). The black suspension is cooled down to room temperature and filtered on a Schott funnel. The light yellow clear filtrate is further filtered through a GFA filter and concentrated in vacuum. The resulting light yellow transparent viscous oil of medetomidine base crystallizes into a porous light mass after removing ethanol traces in vacuum (0.05 mmHg) at room temperature for 20 hours.

**Yield 5.85 g (86%).** ¹H NMR (400 MHz, CDCl₃) δ 10.56 (s, 1H), 7.30-6.62 (m, 5H), 4.35 (q, 1H), 2.26 (s, 3H), 2.17 (s, 3H), 1.56 (d, 3H); ¹³C (100 MHz, CDCl₃) δ 143.38, 141.31, 136.87, 134.60, 134.16, 128.04, 125.65, 124.77, 117.26, 34.23, 21.01, 20.85, 14.76; tR 3.12 min (HPLC Area 98.0%, method C).

### Medetomidine 2 (Route C)

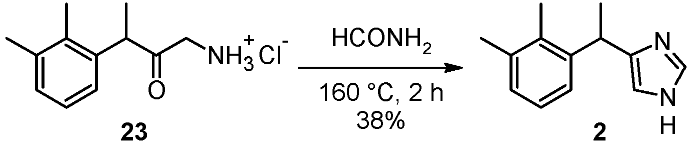

1.29 g (5.66 mmol) of 1-amino-3-(2,3-dimethylphenyl)butan-2-one hydrochloride **23** and 2.55 g (56.6 mmol; 10 eq) of formamide are weighed in a 30 ml glass vial. The vial is purged with argon, a magnetic anchor is placed inside, capped, and the reaction mixture is heated at 160°C for 2 hours (analysis of an aliquot of the reaction mixture after this time shows >99% conversion of starting compound **23**). The reaction mixture is cooled down to room temperature and diluted with 9.63 ml of 36% hydrochloric acid. The homogeneous transparent yellow solution is stirred on a magnetic stirrer at room temperature for 1 hour to hydrolyze the excessive formamide, the precipitated ammonium chloride suspension is diluted with 10 ml of deionized water, and the aqueous phase is extracted with 2×5 ml of ethyl acetate to remove minor impurities. The aqueous phase is cooled down to -3...-5°C and neutralized with 12 ml of 25% aqueous ammonia solution. The separated oil is extracted with 2×10 ml of ethyl acetate, the combined organic extract is washed with 10 ml of deionized water, dried over anhydrous sodium sulfate, filtered and concentrated in vacuum. The obtained base of medetomidine in the form of light yellow oil foams in a vacuum, and after complete removal of ethyl acetate traces, it crystallizes in the form of a bulk porous cream-colored mass.

The product can be purified by crystallization from a cyclohexane-toluene (9:1 v/v) mixture, or converted to its hydrochloride by the standard procedure described above.

**Yield 0.43 g (38%).** ¹H NMR (400 MHz, CDCl₃) δ 10.56 (s, 1H), 7.30-6.60 (m, 5H), 4.36 (q, 1H), 2.27 (s, 3H), 2.18 (s, 3H), 1.56 (d, 3H); ¹³C (100 MHz, CDCl₃) δ 143.38, 141.31, 136.87, 134.60, 134.16, 128.04, 125.65, 124.77, 117.26, 34.23, 21.01, 20.85, 14.76; tR 3.12 min (HPLC Area 95.2%, method C).

## Claims

1. Synthetic method of starting compound **5** to obtain medetomidine and its derivatives, including cobalt-catalyzed cross-coupling of substituted aryl magnesium halide **10** 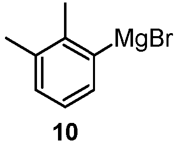 with carbonyl-protected 3-bromo-2-butanone **14** 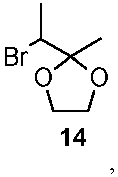 one pot deprotection to obtain 3-(2,3-dimethylphenyl)butan-2-one **7** 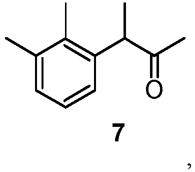 followed by halogenation of 3-(2,3-dimethylphenyl)butan-2-one **7** with molecular bromine to obtain the starting 1-bromo-3-(2,3-dimethylphenyl)butan-2-one **5**

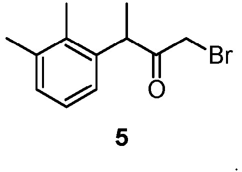

2. Method for obtaining a medetomidine derivative in the form of the copper (I) complex of formula 3 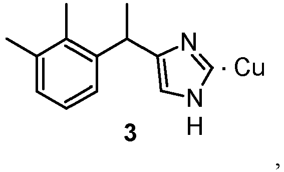 including the interaction of starting compound **5** according to claim 1 with potassium acetate to obtain 3-(2,3-dimethylphenyl)-2-oxobutyl acetate **4** 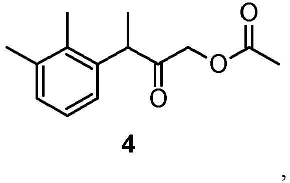 and a multicomponent reaction of compound **4** with formaldehyde and copper (II) acetate in an water-alcohol ammonia solution.

3. Method for obtaining medetomidine of formula **2** 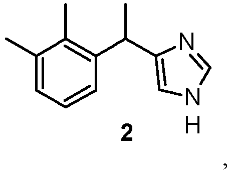 wherein medetomidine base **2** is isolated from the copper (I) complex **3** according to claim 2 by reacting compound **3** with the pentasodium salt of diethylenetriamine pentaacetic acid (NasDTPA).

4. Method of claim 3, wherein pharmaceutically acceptable salts of medetomidine **2** are prepared by reacting the free base of medetomidine **2** with acids in a suitable organic solvent, e.g. medetomidine hydrochloride **1** 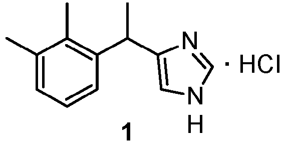 by reaction of the free base of medetomidine **2** with hydrochloric acid in acetone.

5. Method for producing 4-[1-(2,3-dimethylphenyl)ethyl]-1,3-dihydro-2H-imidazole-2-thione medetomidine derivative of formula **24** 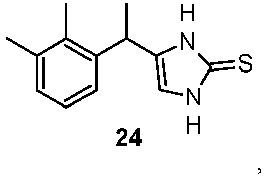 including the interaction of starting compound **5** according to claim 1 with sodium diformylamide **21** 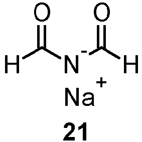 to obtain N-[3-(2,3-dimethylphenyl)-2-oxobutyl]-N-formylformamide **22** 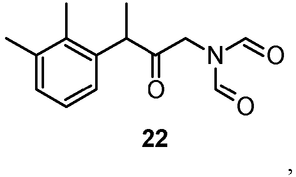 acid hydrolysis of compound **22** to obtain 1-amino-3-(2,3-dimethylphenyl)butan-2-one hydrochloride **23** 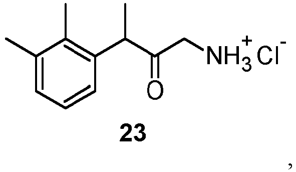 and reaction of compound 23 with potassium thiocyanate.

6. Method for obtaining medetomidine of formula **2** 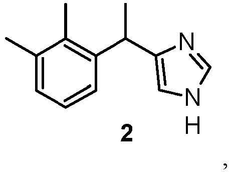 including desulfurization of derivative **24** according to claim 5.

7. Method for obtaining medetomidine of formula **2** 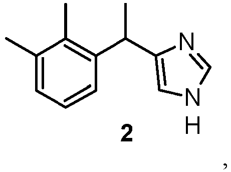 including the interaction of starting compound **5** according to claim 1 with sodium diformylamide **21** to obtain N-[3-(2,3-dimethylphenyl)-2-oxobutyl]-N-formylformamide **22,** acid hydrolysis of compound **22** to obtain 1-amino-3-( 2,3-dimethylphenyl)butan-2-one hydrochloride **23,** and its interaction with formamide.
